# EUROPEAN PATENT APPLICATION

(11) **EP 4 549 545 A1**
(43) Date of publication of application: **07.05.2025**
(21) Application number: 23936786.5
(22) Date of filing: 18.10.2023
(51) Int. Cl.: C12M 1/12, C12N 5/00, C12N 5/077, A23L 13/00

(54) **CULTIVATED MEAT SCAFFOLD, METHOD FOR PRODUCING SAME, AND CULTIVATED MEAT CONTAINING SAME**

(30) Priority: 19.09.2023 KR 20230124967
(71) Applicant: Anpoly Inc., Gyeongsangbuk-do 37673 (KR)
(72) Inventor: RHO, Sang Cheol, Pohang-si Gyeongsangbuk-do 37685 (KR)
(74) Representative: Potter Clarkson
(86) International application number: PCT/KR2023/016112
(87) International publication number: WO 2025/063362

(57) **Abstract**

The present disclosure relates to a cultured meat scaffold, a method of preparing the same, and cultured meat including the same. The cultured meat scaffold according to the present disclosure includes a nanocellulose; and a polymer hydrogel.

## Description

### TECHNICAL FIELD

The present disclosure relates to a cultured meat scaffold, a method of preparing the same, and cultured meat including the same.

### BACKGROUND ART

Cultured meat is produced from cell culture using tissue engineering techniques and is a promising alternative to traditional meat production using live animals. Meat produced from animals has issues with the use of a large number of consumable resources including water, grain, land, and energy for the growth of livestock, especially cattle, and high greenhouse gas emissions. In contrast, cultured meat uses less energy and resources and may also reduce greenhouse gas emissions. In addition, intensive factory farming and poor animal welfare conditions for meat production may cause food poisoning, and growth hormones and antibiotics are administered to a large number of farm animals, which are ultimately passed on to consumers. On the other hand, producing meat in a sterile environment may help improve a food safety. Meat produced under strict regulations and controlled conditions is less likely to transmit contaminants and is safer for consumption. In addition, the nutritional value of meat may be enhanced and made healthier by controlling protein content, fat content, and composition, and essential trace elements and vitamins including iron, zinc, and the like. However, it is a difficult task to increase product safety while improving characteristics of meat products, such as visual characteristics, nutritional characteristics, taste, or flavor.

In general, cell growth is achieved through a series of processes such as cell attachment, proliferation, differentiation, and organization. To prepare cultured meat, it is essential for cells to be organized, and through such an organization, a texture and nutrition become similar to those of actual meat. However, when cells are cultured in a general two-dimensional cell culture dish, it is difficult to organize the cells, and a scaffold on which cells may be attached and grown in the preparation of cultured meat temporarily or permanently has an influence on a cell growth and is an essential element that enables a three-dimensional organization of cells. Nevertheless, materials used as existing cultured meat scaffolds have limitations of low mechanical properties, which makes it impossible to maintain the shape in a cell culture environment when scaffolds are prepared. To solve such an issue, a method of increasing physical properties of a scaffold by adding a chemical cross-linking agent may be provided, but this may cause cytotoxicity. Therefore, there is a need to develop a scaffold that may implement a texture similar to that of actual meat without a chemical cross-linking agent, minimize cytotoxicity and the burden of ingestion, and enable cells to be easily and quickly cultured.

The above description has been possessed or acquired by the inventor(s) in the course of conceiving the present disclosure and is not necessarily an art publicly known before the present application is filed.

### DISCLOSURE OF THE INVENTION

### TECHNICAL GOALS

To solve the above-described problems, the present disclosure is to provide a cultured meat scaffold with excellent physical properties and excellent cell culture activity, a method of preparing the same, and cultured meat including the same.

However, goals to be achieved by the present disclosure are not limited to those described above, and other goals not mentioned above can be clearly understood by one of ordinary skill in the art from the following description.

### TECHNICAL SOLUTIONS

A cultured meat scaffold according to the present disclosure includes a nanocellulose; and a polymer hydrogel.

According to an embodiment, the nanocellulose may be in a form of a powder, a suspension, or a hydrogel.

According to an embodiment, the nanocellulose may have a diameter of 1 nanometer (nm) to 10 micrometers (µm).

According to an embodiment, the nanocellulose may be a hydrogel having a viscosity of 12,000 centipoise (cP) or greater, and a gelation time is in a range of 1 second to 30 seconds.

According to an embodiment, endotoxin level in the nanocellulose may be 5 endotoxin units per milliliter (EU/mL) or less.

According to an embodiment, the polymer hydrogel may include at least one selected from a group consisting of collagen, chitosan, fibrin, agar, starch, alginate, hyaluronic acid, gelatin, dextran, elastin, carrageenan, polyethylene glycol, polyacrylic acid, polyvinyl alcohol, polyvinylpyrrolidone, polylactic acid, and polyglycolic acid.

According to an embodiment, the polymer hydrogel may be in a form of a bead or bulk.

According to an embodiment, a weight ratio of the nanocellulose to the polymer hydrogel may be in a range of 1 : 4 to 3 : 2.

According to an embodiment, the scaffold may have a pore diameter of 5 µm to 500 µm, a porosity of 70% to 95%, and a pore uniformity of 10% to 40%.

According to an embodiment, the scaffold may have a density of 1 gram per cubic centimeter (g/cm³) to 10 g/cm³, and a strength of 0.1 megapascal (MPa) to 20 MPa, and endotoxin level may be 5 EU/mL or less in the scaffold.

A method of preparing a cultured meat scaffold according to the present disclosure includes a step of preparing a nanocellulose; a step of mixing the nanocellulose with a polymer hydrogel; and a step of performing freeze-drying.

According to an embodiment, the step of preparing the nanocellulose may include a step of treating a cellulose at a temperature of 20°C to 50°C for 30 minutes to 3 hours; a step of performing washing; and a step of performing a nanotization.

According to an embodiment, the step of performing the freeze-drying may include performing the freeze-drying at a temperature of -50°C to -80°C for 48 hours to 72 hours.

A cultured meat according to the present disclosure is prepared by culturing a cell or a tissue on the cultured meat scaffold according to the present disclosure or a cultured meat scaffold prepared by the method of preparing the cultured meat scaffold according to the present disclosure.

According to an embodiment, a three-dimensional (3D) structure of the scaffold may be maintained during the culturing of the cell or tissue.

According to an embodiment, the cell may include at least one selected from a group consisting of a muscle stem cell, a bone cell, a muscle cell, a fat cell, and a stem cell.

According to an embodiment, a cell or tissue viability after the culturing may be greater than or equal to 80%, and a culture activity may be greater than or equal to 20%.

### EFFECTS OF THE INVENTION

The present disclosure may provide a cultured meat scaffold having excellent physical properties and excellent cell culture activity, a method of preparing the same, and cultured meat including the same.

Specifically, the cultured meat scaffold according to the present disclosure may include a nanocellulose and a polymer hydrogel, to be free of cytotoxicity, and a pore size and density may be controlled to enhance physical properties of the scaffold, thereby exhibiting an excellent culture activity of cells or tissues during a preparation of cultured meat, and thus, it is possible to easily and quickly provide cultured meat with a texture similar to that of meat.

### BRIEF DESCRIPTION OF DRAWINGS

FIGS. 1A and 1B illustrate images representing cultured meat according to an example and a comparative example of the present disclosure.
FIGS. 2A and 2B illustrate images representing cultured meat scaffolds according to an example and a comparative example of the present disclosure.
FIGS. 3A and 3B illustrate scanning electron microscope (SEM) images of cultured meat scaffolds according to an example and a comparative example of the present disclosure.
FIGS. 4A and 4B illustrate images for verifying cultures of cells on cultured meat scaffolds according to an example and a comparative example of the present disclosure.
FIG. 5 is a graph illustrating a cell viability according to an example of the present disclosure.

### BEST MODE FOR CARRYING OUT THE INVENTION

Hereinafter, embodiments will be described in detail with reference to the accompanying drawings. However, various alterations and modifications may be made to the embodiments, and the embodiments are not meant to be limited by the descriptions of the present disclosure. The embodiments should be understood to include all changes, equivalents, and replacements within the idea and the technical scope of the disclosure.

The terminology used herein is for the purpose of describing particular embodiments only and is not intended to be limiting. The singular forms "a," "an," and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

In addition, when describing the embodiments with reference to the accompanying drawings, like reference numerals refer to like components and a repeated description related thereto will be omitted. In the description of embodiments, detailed description of well-known related structures or functions will be omitted when it is deemed that such description will cause ambiguous interpretation of the present disclosure. In addition, terms such as first, second, A, B, (a), (b), and the like may be used to describe components of the embodiments. Each of these terms is not used to define an essence, order or sequence of a corresponding component but used merely to distinguish the corresponding component from other component(s). It should be understood that if it is described in the specification that one component is "connected," "coupled," or "joined" to another component, the former may be directly "connected," "coupled," and "joined" to the latter or "connected," "coupled," and "joined" to the latter via another component.

A component, which has the same common function as a component included in any one embodiment, will be described by using the same name in other embodiments. Unless disclosed to the contrary, the description of any one embodiment may be applied to other embodiments, and the specific description of the repeated configuration will be omitted.

A cultured meat scaffold according to the present disclosure includes nanocellulose; and polymer hydrogel.

The cultured meat scaffold according to the present disclosure may provide a surface and a support for cell attachment, maturation, differentiation, and proliferation to prepare cultured meat, and may remove limitations of materials used as cultured meat scaffolds in a related art, such as materials with low mechanical properties that make it impossible to maintain a shape in a cell culture environment or materials with low cell adhesion that make cell growth difficult when only a single material is used. In other words, since the nanocellulose and the polymer hydrogel are included, a pore size and density may be controlled, thereby enhancing physical properties of the scaffold and metabolism of increased waste excretion and nutrient supply, and thus, cultured meat with an excellent texture due to a high culture activity may be provided.

The nanocellulose may be easily mixed with the polymer hydrogel due to its high miscibility, thereby compensating for low mechanical properties of a hydrogel.

The cultured meat scaffold may further include nanochitin or a catechol-conjugated levan.

According to an embodiment, the nanocellulose may be in a form of a powder, a suspension, or a hydrogel.

The nanocellulose according to the present disclosure may be in the form of a powder, a suspension, or a hydrogel and a suitable form of nanocellulose may be selected as necessary.

The nanocellulose is a polymer material obtained by splitting a cellulose into nanosized pieces by one billionth, five times stronger and five times lighter than iron, flexible, and environmentally friendly, and has safe antibacterial properties.

According to an embodiment, the nanocellulose may have a diameter of 1 nanometer (nm) to 10 micrometers (µm).

Desirably, the diameter of the nanocellulose may be in a range of 1 nm to 1 µm; 1 nm to 500 nm; 1 nm to 100 nm; 1 nm to 50 nm; 50 nm to 10 µm; 50 nm to 1 µm; 50 nm to 500 nm; 50 nm to 100 nm; 100 nm to 10 µm; 100 nm to 1 µm; 100 nm to 500 nm; 500 nm to 10 µm; 500 nm to 1 µm; or 1 µm to 10 µm.

If the diameter falls below the above range, a mechanical strength may decrease and it may be difficult to maintain a structure, and if the diameter exceeds the above range, the effect of the scaffold may be reduced due to an increase in pores, a decrease in a specific surface area, and the like.

According to an embodiment, the nanocellulose may be a hydrogel having a viscosity of 12,000 centipoise (cP) or greater, and a gelation time may be in a range of 1 second to 30 seconds.

If the nanocellulose is in the form of a hydrogel, the viscosity may be 12,000 cP or greater. If the viscosity falls below the above range, a scaffold may not be formed normally. If the viscosity is greater than or equal to the above viscosity, there is an advantage in that the structure may be stably maintained even under various environmental conditions.

The gelation time of the nanocellulose may be desirably in a range of 1 second to 20 seconds, and more desirably in a range of 1 second to 10 seconds. A nanocellulose according to the related art has issues of not gelating or needing to add a specific material for gelation, however, the nanocellulose according to the present disclosure does not require a separate additive due to a short gelation time, may shorten a time to prepare a scaffold, and in particular, may prevent contamination caused by long-term exposure.

According to an embodiment, endotoxin level in the nanocellulose may be 5 endotoxin units per milliliter (EU/mL) or less.

Endotoxin is a toxic substance that is a powerful pyrogen and may be an indicator of a biological safety. In the cultured meat scaffold according to the present disclosure, cells may be cultured on a scaffold to produce cultured meat and consume the cultured meat, and it is necessary to ensure the biological safety. If the endotoxin is less than or equal to the above range, a cell viability may be high during a preparation of cultured meat, because there is no cytotoxicity, and it may be harmless to human bodies to be suitable as an edible cultured meat scaffold.

According to an embodiment, the nanocellulose may have a concentration of 0.1 milligrams per milliliter (mg/ml) to 10 mg/mL.

The concentration of the nanocellulose may be properly selected and used, however, if the concentration of the nanocellulose in the scaffold is in the above range, a scaffold with excellent physical properties may be prepared.

According to an embodiment, the polymer hydrogel may include at least one selected from a group consisting of collagen, chitosan, fibrin, agar, starch, alginate, hyaluronic acid, gelatin, dextran, elastin, carrageenan, polyethylene glycol, polyacrylic acid, polyvinyl alcohol, polyvinylpyrrolidone, polylactic acid, and polyglycolic acid.

Hydrogels contain a large amount of water and have properties between liquids and solids and polymer chains thereof form a three-dimensional (3D) structure and absorb a large amount of water to swell, but the hydrogels do not dissolve in water due to their crosslinked structure and may mimic tissues of actual organisms. The polymer hydrogel may be used without a limitation as long as it is a polymer that may be used to prepare cultured meat. The polymer hydrogel has an advantage of being easy to form a structure due to a simple process of dissolving and shaping a material, and thus, the polymer hydrogel may be used as a 3D cell culture scaffold by removing an issue of two-dimensional (2D) cell culture creating an environment different from that of actual tissue in a process of preparing cultured meat.

According to an embodiment, the polymer hydrogel may be in a form of a bead or bulk.

The polymer hydrogel according to the present disclosure may be in the form of a bead or bulk, and a suitable form of the polymer hydrogel may be selected as necessary.

According to an embodiment, a weight ratio of the nanocellulose to the polymer hydrogel may be in a range of 1 : 4 to 3 : 2.

Desirably, the weight ratio of the nanocellulose to the polymer hydrogel may be in a range of 1 : 4 to 5 : 4; 1 : 4 to 1 : 1; 1 : 4 to 3 : 4; 1 : 4 to 1 : 2; 1 : 2 to 3 : 2; 1 : 2 to 5 : 4; 1 : 2 to 1 : 1; 1 : 2 to 3 : 4; 3 : 4 to 3 : 2; 3 : 4 to 5 : 4; 3 : 4 to 1 : 1; 1 : 1 to 3 : 2; 1 : 1 to 5 : 4; or 5 : 4 to 3 : 2.

A pore size and density of the scaffold may be controlled based on a mixing ratio of the nanocellulose and the polymer hydrogel, and when the nanocellulose and the polymer hydrogel are mixed at a weight ratio within the above-mentioned range, an optimal pore size, density, and pore uniformity may be exhibited so that a scaffold having excellent physical properties may be prepared.

According to an embodiment, the scaffold may have a pore diameter of 5 µm to 500 µm, a porosity of 70% to 95%, and a pore uniformity of 10% to 40%.

The scaffold may form pores by mixing a nanocellulose and a polymer hydrogel. The pores may control the physical properties of the scaffold in association with the proliferation, differentiation, and attachment of cultured meat cells. The cultured meat scaffold according to the present disclosure may increase a uniformity of pores and properly maintain the shape of pores due to an addition of the nanocellulose, and thus, metabolism, i.e., nutrient supply and waste excretion may increase.

The pore diameter may refer to a maximum length passing through the center of a pore. Desirably, the pore diameter may be in a range of 5 µm to 400 µm; 5 µm to 300 µm; 5 µm to 200 µm; 5 µm to 100 µm; 50 µm to 500 µm; 50 µm to 400 µm; 50 µm to 300 µm; 50 µm to 200 µm; 50 µm to 100 µm; 100 µm to 500 µm; 100 µm to 400 µm; 100 µm to 300 µm; 100 µm to 200 µm; 200 µm to 500 µm; 200 µm to 400 µm; 200 µm to 300 µm; 300 µm to 500 µm; 300 µm to 400 µm; or 400 µm to 500 µm.

The porosity may desirably be in a range of 70% to 90%; 70% to 85%; 70% to 80%; 70% to 75%; 75% to 95%; 75% to 90%; 75% to 85%; 75% to 80%; 80% to 95%; 80% to 90%; 80% to 85%; 85% to 95%; 85% to 90%; or 90% to 95%.

The pore uniformity may refer to a difference between an average pore size and a pore size of each pore, and the pore size may be greater or less by 10% to 40% than the average pore size. Desirably, it may be in a range of 10% to 30%; 10% to 20%; 20% to 40%; 20% to 30%; or 30% to 40%.

If the pore diameter falls below the above range, an issue of a decrease in a cell attachment rate may occur, and if the pore diameter exceeds the above range, an issue of failing to function as a support during a cell culture may occur.

If the porosity falls below the above range, an issue of inhibiting a cell growth may occur, and if the porosity exceeds the above range, an issue of failing to function as a support during a cell culture may occur.

If the pore uniformity exceeds the above range, an issue of a decrease in reproducibility during a cell culture may occur.

When the scaffold has an appropriate pore size, porosity, and pore uniformity, a shape of the scaffold may be stably maintained during the cell culture, cells may more easily penetrate into the scaffold, and a nutrient circulation and oxygen supply may increase, to provide an optimal environment for a cell growth.

According to an embodiment, the scaffold may have a density of 1 gram per cubic centimeter (g/cm³) to 10 g/cm³, and a strength of 0.1 megapascal (MPa) to 20 MPa, and endotoxin level may be 5 EU/mL or less in the scaffold.

Since the scaffold includes a mixture of the nanocellulose and the polymer hydrogel, the scaffold with excellent physical properties may be provided. The physical properties of the scaffold may be expressed as a density, a strength, a toughness, a rigidity, a cell viability, and the like, the density may desirably be in a range of 1 g/cm³ to 8 g/cm³; 1 g/cm³ to 5 g/cm³; 1 g/cm³ to 3 g/cm³; 3 g/cm³ to 10 g/cm³; 3 g/cm³ to 8 g/cm³; 3 g/cm³ to 5 g/cm³; 5 g/cm³ to 10 g/cm³; or 5 g/cm³ to 8 g/cm³, and the strength may desirably be in a range of 0.1 MPa to 10 MPa; 0.1 MPa to 5 MPa; 0.1 MPa to 1 MPa; 0.1 MPa to 0.5 MPa; 0.5 MPa to 20 MPa; 0.5 MPa to 10 MPa; 0.5 MPa to 5 MPa; 0.5 MPa to 1 MPa; 1 MPa to 20 MPa; 1 MPa to 10 MPa; 1 MPa to 5 MPa; 5 MPa to 20 MPa; 5 MPa to 10 MPa; or 10 MPa to 20 MPa.

If the density falls below the above range, a bond between cells may not be formed during a culture, which may result in a decrease in a texture, and if the density exceeds the above range, a space in which cells are to differentiate during a culture may be reduced, which may lead to an issue of cells failing to sufficiently mature. The strength may be a compressive strength, if the strength falls below the above range, the scaffold may fail to function as a support and collapse, and if the strength exceeds the above range, cells may fail to stably grow. When the physical properties are within the above ranges, cells may be stably, easily, and quickly cultured, and a cell viability during a cell culture may be greater than or equal to 80%.

The cultured meat scaffold according to the present disclosure may exhibit an optimal density and strength by adding the nanocellulose, and thus, the cultured meat scaffold may not easily collapse even when a physical tensile force is applied. This may help improve a texture of cultured meat by enabling cells to be aligned and differentiated by applying a tensile force when cultured meat is prepared.

Since cultured meat needs to be prepared and consumed, the cultured meat scaffold necessarily require a biological safety. The safety may be determined through endotoxin that is a toxic substance, and if the endotoxin falls below the above range, a cell viability may be high, because there is no cytotoxicity, and it may be harmless to human bodies to be suitable as an edible cultured meat scaffold.

A method of preparing a cultured meat scaffold according to the present disclosure includes a step of preparing a nanocellulose; a step of mixing the nanocellulose with a polymer hydrogel; and a step of performing freeze-drying.

Since the cultured meat scaffold according to the present disclosure is prepared by mixing the nanocellulose and the polymer hydrogel, a safe scaffold with excellent physical properties may be provided, and thus, it is possible to prepare cultured meat with an excellent texture.

According to an embodiment, the step of preparing the nanocellulose may include a step of treating a cellulose at a temperature of 20°C to 50°C for 30 minutes to 3 hours; a step of performing washing; and a step of performing a nanotization.

The cellulose may be obtained by pretreating, such as defibrating, wood-based or non-wood-based raw materials having 70% or greater of cellulose using a pulper or refinery.

The step of treating the cellulose may be treating a pretreated cellulose with TEMPO or an enzyme, or reacting an untreated cellulose under the above conditions. The step of treating the cellulose may be performed desirably for 30 minutes to 2 hours, and more desirably for 1 hour to 2 hours.

If the temperature and time in the step of treating the cellulose are beyond the above ranges, an issue may occur in preparing of a nanocellulose having a mechanical strength and viscosity suitable for a scaffold.

The step of performing washing may desirably be performed at least two times.

The step of performing the nanotization may be performing treating at least once and a nanotization using nanotization equipment, such as a high-pressure homogenizer or an ultrasonic emulsifier.

Cellulose has a large diameter, a small surface area, and poor uniformity among fibers, which may make it difficult to prepare a scaffold having a uniform pore size and porosity, however, a nanocellulose obtained by the method according to the present disclosure has an extremely small diameter, a large surface area, and extremely high uniformity among fibers, as well as a high porosity, which may lead to a smooth supply of oxygen and nutrients to cells, thereby making it possible to prepare a uniform and stable support for cell adhesion and growth.

According to an embodiment, the step of performing the freeze-drying may be performing the freeze-drying at a temperature of -50°C to -80°C for 48 hours to 72 hours.

If a temperature and time for the freeze-drying is less than the above ranges, it may not be completely dried, which may cause an issue of unstable or uneven shape, and if the temperature and time for the freeze-drying is beyond the above range, an issue of a difference in physical properties between a surface and an interior due to excessive drying of the surface may occur. If the temperature and time for the freeze-drying is within the above range, moisture may be completely removed, and physical properties and structures of the surface and the interior may be identical.

Cultured meat according to the present disclosure may be prepared by culturing a cell or tissue on the cultured meat scaffold according to the present disclosure or a cultured meat scaffold prepared by the method of preparing the cultured meat scaffold according to the present disclosure.

In the related art, cultured meat has an issue of a difficulty to obtain a texture and product safety. In the cultured meat according to the present disclosure, cells or tissues may be cultured on a cultured meat scaffold including a nanocellulose and a polymer hydrogel, culturing may be easily and quickly performed due to high cell or tissue culture activities, and the supply of nutrients and excretion of waste products in a culture process may be excellent, and thus, it is possible to obtain cultured meat with excellent texture and safety.

The culturing may be seeding or printing cells or tissues.

According to an embodiment, a 3D structure of the scaffold may be maintained during the culturing of the cell or tissue.

In the related art, a cell culture has been performed using a 2D cell culture method using a petri dish. However, when the 2D cell culture method is used to culture cells, there is an issue of creating an environment different from that of an actual tissue, such as an issue of changing the shape of cells, or an issue of a possibility of changing a gene expression even when cell division is performed. Therefore, methods to culture cells similarly to actual tissues using a 3D cell culture method are being studied, and accordingly, a cell attachment scaffold having a structure similar to characteristics of an extracellular matrix is important. Since a scaffold prepared using only a single material has extremely low cell adhesion to be used as a cell culture scaffold, it is difficult to effectively grow cells. The cultured meat according to the present disclosure may be cultured while maintaining a 3D structure by forming a mesh structure by inducing a hydrogen bond between hydroxyl groups on a surface of a nanocellulose using a cultured meat scaffold including a nanocellulose and a polymer hydrogel, and thus, excellent cultured meat may be prepared by increasing a cell or tissue viability and culture activity due to an effective cell growth.

According to an embodiment, the cell may include at least one selected from a group consisting of a muscle stem cell, a bone cell, a muscle cell, a fat cell, and a stem cell.

The cells may be cells derived from non-human animals and are not limited to cells that may be used to prepare cultured meat, but may desirably be muscle stem cells.

According to an embodiment, a cell or tissue viability after the culturing may be greater than or equal to 80%, and a culture activity may be greater than or equal to 20%.

In the cultured meat according to the present disclosure, cells or tissues may be cultured using a scaffold including a nanocellulose and a polymer hydrogel, and a culture activity and a cell or tissue viability during the culturing may be excellent, and thus, cultured meat with an excellent texture may be easily and quickly formed.

The above viability may be a ratio of the number of surviving cells after culturing to the number of surviving cells before culturing.

In the related art, it is impossible to maintain a shape due to low physical properties of a scaffold, or a viability is low because the metabolism fails to be smoothly performed due to a decrease in a cell adhesion, however, the cultured meat according to the present disclosure may have a low cytotoxicity and an excellent cell adhesion of the scaffold, and thus, cultured meat with excellent physical properties while exhibiting a high viability and culture activity greater than or equal to the above range may be provided.

A cultured meat scaffold according to the present disclosure may include a nanocellulose and a polymer hydrogel, and a scaffold having excellent physical properties and an optimal pore size and density may be prepared according to a ratio of the nanocellulose to the polymer hydrogel according to the present disclosure, and thus, cultured meat that has an excellent texture and that is free of cytotoxicity may be easily and quickly prepared due to excellent cell or tissue culture activity and viability when cultured meat is prepared.

Hereinafter, the present disclosure will be described in detail with reference to the following example and comparative example.

However, the following example is only for illustrating the present disclosure, and the description of the present disclosure is not limited to the following example.

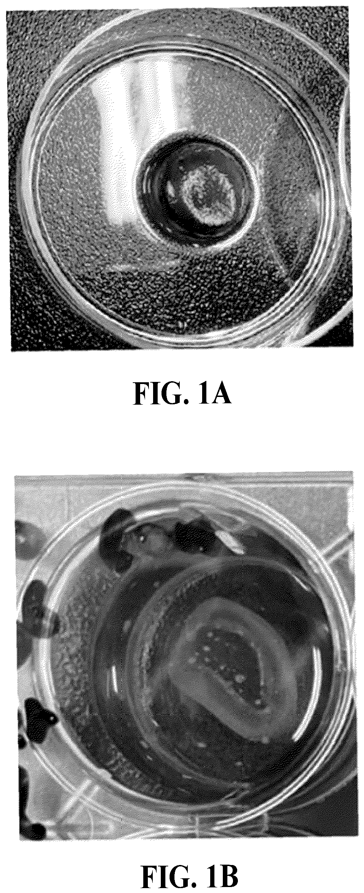

FIGS. 1A and 1B illustrate images representing cultured meat according to an example and a comparative example of the present disclosure. Referring to FIG. 1A, it can be confirmed that cultured meat according to the present disclosure has a high density and tense tissues and confirmed that in the comparative example of FIG. 1B, the shape is unstable and tissues are extremely loose.

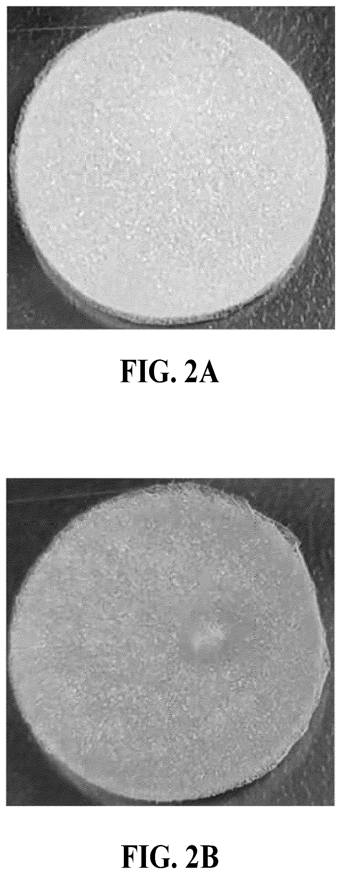

FIGS. 2A and 2B illustrate images representing cultured meat scaffolds according to an example and a comparative example of the present disclosure. Referring to FIGS. 2A and 2B, comparing the example of FIG. 2A and the comparative example of FIG. 2B, it can be confirmed that the cultured meat scaffold in the example has a relatively high density and uniform pores.

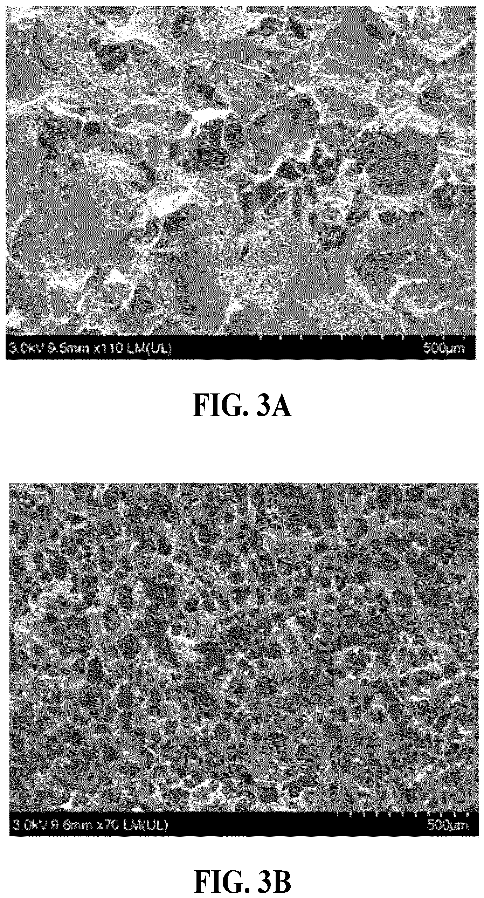

FIGS. 3A and 3B illustrate scanning electron microscope (SEM) images of cultured meat scaffolds according to an example and a comparative example of the present disclosure. FIG. 3A illustrates a scaffold that does not include nanocellulose, and FIG. 3B illustrates a scaffold with a nanocellulose and a polymer hydrogel in a weight ratio of 2.5 : 7.5. Referring to FIGS. 3A and 3B, it can be confirmed that the pore size decreases due to an addition of nanocellulose and found that pores are uniform.

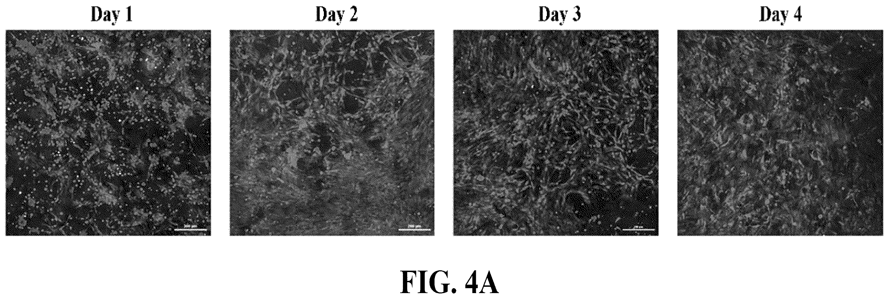

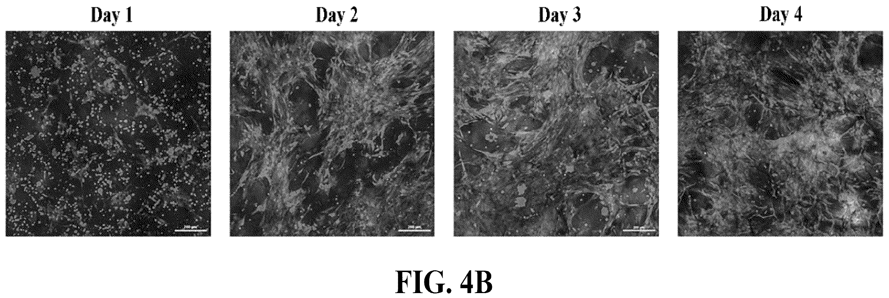

FIGS. 4A and 4B illustrate images for verifying cultures of cells on cultured meat scaffolds according to an example and a comparative example of the present disclosure. FIG. 4A illustrates results of the comparative example, and FIG. 4B illustrates results of the example. In the case of a cultured meat scaffold developed through this, it can be confirmed that a cell growth rate is high during the same culture period and that cells grow evenly throughout the scaffold.

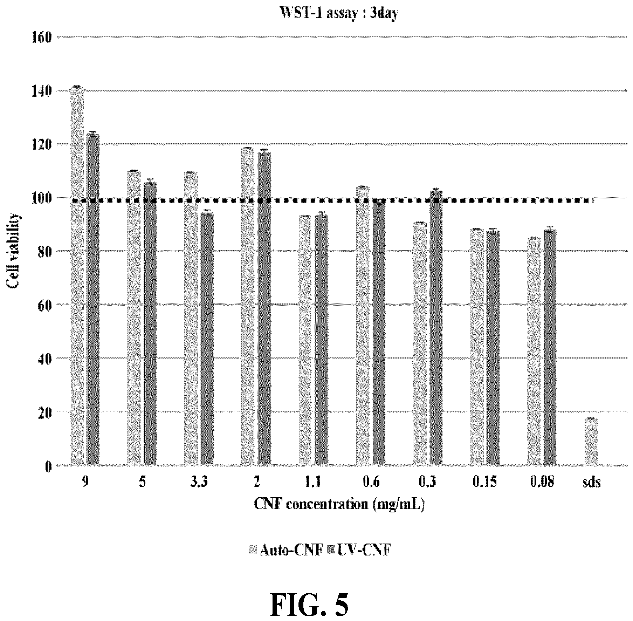

FIG. 5 is a graph illustrating a cell viability according to an example of the present disclosure. To identify a cell survival capability, the cell viability was measured using a WST-1 assay, and it can be confirmed that the cultured meat prepared using the scaffold prepared according to the present disclosure has an excellent cell viability and found that as the concentration increases, the cell viability also increases.

While the embodiments are described with reference to drawings, it will be apparent to one of ordinary skill in the art that various alterations and modifications in form and details may be made in these embodiments without departing from the spirit and scope of the claims and their equivalents. For example, suitable results may be achieved if the described techniques are performed in a different order and/or if components in a described system, architecture, device, or circuit are combined in a different manner and/or replaced or supplemented by other components or their equivalents.

Therefore, other implementations, other embodiments, and equivalents to the claims are also within the scope of the following claims.

## Claims

1. A cultured meat scaffold comprising:
a nanocellulose; and
a polymer hydrogel.

2. The cultured meat scaffold of claim 1, wherein the nanocellulose is in a form of a powder, a suspension, or a hydrogel.

3. The cultured meat scaffold of claim 1, wherein the nanocellulose has a diameter of 1 nanometer (nm) to 10 micrometers (µm).

4. The cultured meat scaffold of claim 1, wherein
the nanocellulose is a hydrogel having a viscosity of 12,000 centipoise (cP) or greater, and
a gelation time is in a range of 1 second to 30 seconds.

5. The cultured meat scaffold of claim 1, wherein endotoxin level in the nanocellulose is 5 endotoxin units per milliliter (EU/mL) or less.

6. The cultured meat scaffold of claim 1, wherein the polymer hydrogel comprises at least one selected from a group consisting of collagen, chitosan, fibrin, agar, starch, alginate, hyaluronic acid, gelatin, dextran, elastin, carrageenan, polyethylene glycol, polyacrylic acid, polyvinyl alcohol, polyvinylpyrrolidone, polylactic acid, and polyglycolic acid.

7. The cultured meat scaffold of claim 1, wherein the polymer hydrogel is in a form of a bead or bulk.

8. The cultured meat scaffold of claim 1, wherein a weight ratio of the nanocellulose to the polymer hydrogel is in a range of 1 : 4 to 3 : 2.

9. The cultured meat scaffold of claim 1, wherein the scaffold has a pore diameter of 50 µm to 500 µm, a porosity of 70% to 95%, and a pore uniformity of 10% to 40%.

10. The cultured meat scaffold of claim 1, wherein
the scaffold has a density of 1 gram per cubic centimeter (g/cm³) to 10 g/cm³, and a strength of 0.1 megapascal (MPa) to 20 MPa, and endotoxin level is 5 EU/mL or less in the scaffold.

11. A method of preparing a cultured meat scaffold, the method comprising:
a step of preparing nanocellulose;
a step of mixing the nanocellulose with a polymer hydrogel; and
a step of performing freeze-drying.

12. The method of claim 11, wherein the step of preparing the
nanocellulose comprises:
a step of treating a cellulose at a temperature of 20°C to 50°C for 30 minutes to 3 hours;
a step of performing washing; and
a step of performing a nanotization.

13. The method of claim 11, wherein the step of preparing the freeze-drying comprises performing the freeze-drying at a temperature of -50°C to -80°C for 48 hours to 72 hours.

14. A cultured meat prepared by culturing a cell or a tissue on the cultured meat scaffold of claim 1, or a cultured meat scaffold prepared by the method of claim 11.

15. The cultured meat of claim 14, wherein a three-dimensional (3D) structure of the scaffold is maintained during the culturing of the cell or tissue.

16. The cultured meat of claim 14, wherein the cell comprises at least one selected from a group consisting of a muscle stem cell, a bone cell, a muscle cell, a fat cell, and a stem cell.

17. The cultured meat of claim 14, wherein
a cell or tissue viability after the culturing is greater than or equal to 80%, and
a culture activity is greater than or equal to 20%.
